# EUROPEAN PATENT SPECIFICATION

(11) **EP 1 350 791 B1**
(45) Date of publication and mention of the grant of the patent: **20.09.2006**
(21) Application number: 03005268.2
(22) Date of filing: 10.03.2003
(51) Int. Cl.: C07D 413/06, C07D 209/20, A23L 1/236

(54) **Process for converting dihydroisoxazole derivatives to gamma-hydroxyamino acid derivatives, e.g. monatins**
Verfahren zur Umwandlung von Dihidroisoxazol Derivate in Gamma-Hydroxaminsäurederivate, z.B Monatins
Procédé de convertion de dihydroisoxazoles en gamma-hydroxyamino acid derivatives, ex. monatins

(30) Priority: 01.04.2002 JP 2002098515
(43) Date of publication of application: 08.10.2003
(73) Proprietor: Ajinomoto Co., Inc., Tokyo 104 (JP)
(72) Inventor: Amino, Yusuke, Kawasaki-shi Kanagawa, 210-0801 (JP); Kawahara, Shigeru, Kawasaki-shi, Kanagawa, 210-0801 (JP)
(74) Representative: Strehl Schübel-Hopf & Partner

(56) References cited:
- US-A- 5 128 482
- HELAINE V ET AL: "Chemo-enzymatic synthesis of the four stereoisomers of 4-hydroxy-4-methylglutamic acid" TETRAHEDRON: ASYMMETRY, ELSEVIER SCIENCE PUBLISHERS, AMSTERDAM, NL, vol. 9, no. 21, 6 November 1998 (1998-11-06), pages 3855-3861, XP004143701 ISSN: 0957-4166

## Description

### FIELD OF THE INVENTION

The present invention relates to a novel process for producing γ-hydroxyamino acid derivatives, especially monatins. More specifically, it relates to a process in which dihydroisoxazole derivatives are subjected to a catalytic hydrogenation reaction under a specific condition to convert the same to γ-hydroxyamino acid derivatives important as various synthetic intermediates, especially to a process in which dihydroisoxazole derivatives having an indolyl group are converted to monatins (including stereoisomers, salts thereof and those with a functional group protected) excellent as sweeteners or active ingredients thereof.

### DESCRIPTION OF THE PELATED ART

In recent years, with the higher level of eating habits, fatness due to excessive intake of sugar in particular and consequent diseases have been at issue, and the development of low-caloric (low-calorie) sweeteners that replace sugar has been in high demand. For these sweeteners, characteristics such as a low calory, a safety, a stability to heat or acid, a sweetness quality and costs have to be taken into consideration in addition to a degree of sweetness (sweetening potency).

Various sweeteners have been currently in use or proposed. As a sweetener with a high degree of sweetness which is capable of industrial mass-production and has actually found wide acceptance, there is, for example, aspartame excellent in safety and sweetness quality. Further, aspartame derivatives have been increasingly studied. Besides them, sweetening materials having various characteristics as sweeteners have been proposed and studied for actual use. Moreover, thaumatin, glycyrrhizin and stevioside derived from plants, which are present in nature and can be collected in large quantities, have been currently used as natural sweeteners.

Monatin is a natural amino acid derivative isolated from the bark of the roots of Schlerochiton ilicifolius, a plant that grows wild in the north-western Transvaal region of South Africa. R. Vleggaar et al. have reported that it is (2S,4S)-2-amino-4-carboxy-4-hydroxy-5-(3-indolyl)pentanoic acid [(2S,4S)-4-hydroxy-4-(3-indolylmethyl)-glutamic acid; see the structural formula (3)][refer to R. Vleggaar et al., J. Chem. Soc. Perkin Trans., 3095 - 3098, (1992)]. Moreover, the degree of sweetness of the (2S,4S) compound derived from the natural plant is determined to be, according to this document, 800 times or 1,400 times that of sucrose. As a process for producing the monatin, some processes have been reported. However, there is no appropriate industrial process [as the process, refer to P. J. van Wyk et al., ZA 87/4288, C. W. Holzapfel et al., Synthetic Communications, 24(22), 3197 - 3211 (1994), E. Abushanab et al., U.S.P. 5,994,559 (1999) and K. Nakamura et al., Organic Letters, 2, 2967 - 2970 (2000)].

C. W. Holzapfel et al. [refer to the above described Synthetic Communications, 24(22), 3197 - 3211 (1994) and U.S.P. 5,128,482 (1992)] reduce dihydroisoxazole derivatives represented by the following structual formula (4) with sodium amalgam (NaHg) to convert the same to monatins represented by the following structural formula (3). However, since this process uses a mercury compound having a high toxicity, it is extremely dangerous in operation. When the products are used as sweeteners, a procedure of thoroughly removing mercury with an ion exchange resin or the like after completion of the reaction is indispensable. The document of U.S.P. 5,128,482 only demonstrates the use of sodium amalgam in Examples in the same way. Further, a "chemically reducing" method is claimed therein as "a method for converting the compounds of the structural formula (4) and the like to the compounds of the structural formula (3) and the like". However, reagents, reaction conditions and the like are neither specifically claimed nor described in detail. In a description part explained for the "chemically reducing" method in claims, sodium amalgam (amalgam reduction), cyanoborohydride (hydride reduction) and sodium (molten metal reduction) are listed as a reducing agent. However, there is nothing to describe the catalytic hydrogenation reaction. It is known that a reduction reaction of an aromatic ring such as an indolyl group proceeds as a side reaction of a catalytic hydrogenation reaction. That is, the hydrogenation reaction for the conversion as described above has not been sufficiently studied.

V. Helaine et al. [refer to Tetrahedron: Asymmetry 9, 3855 - 3861 (1998)] convert a diethyl 5-methyl-4,5-dihydroisoxazole-3,5-dicarboxylate (compound of the general formula (1) in which R¹ is a methyl group and R² and R³ are each an ethyl group) to a γ-hydroxyamino acid derivative. They have reported that when the compound is reacted as such, conversion to a lactam occurs and the reaction is therefore conducted in the presence of benzoic anhydride to obtain an N-benzoyl derivative. Thus, although the γ-hydroxyamino acid derivative is obtained by the catalytic hydrogenation reaction of the derivative in which R¹ is a methyl group, V. Helaine et al. make a heat-reflux to the reaction solution overnight in 6N-hydrochloric acid aqueous solution for removal of the benzoyl group. For example, when a functional group extremely weak to an acid, such as an indolylmethyl group is present in a molecule, such severe deprotection conditions cannot be applied. Thus, a conversion method that does not require such a procedure is preferable.

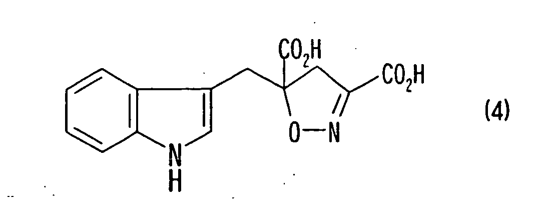

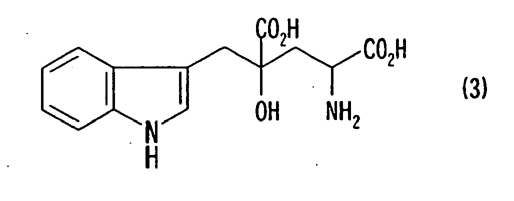

Under these circumstances, the development of a practical and simple process for converting the dihydroisoxazole derivatives represented by the structural formula (4) and the like to the monatins represented by the structural formula (3) has been demanded as a process for producing monatins.

### SUMMARY OF THE TNVENTION

The problem to be solved by the present invention is to develop a convenient process for converting dihydroisoxazole derivatives to γ-hydroxyamino acid derivatives important as various synthetic intermediates and further converting specific dihydroisoxazole derivatives to monatins that can be expected to be put to practical use as sweeteners.

The present inventors have assiduously conducted investigations to solve the problem. That is, they have studied various catalytic hydrogenation reactions on the compounds represented by the general formula (1) or the structural formula (4) described above, especially catalysts, solvents, additives, a hydrogen pressure and the like. Consequently, they have found an appropriate process in which the compounds represented by the general formula (2) or the structural formula (3) described above can be obtained in high yield.

Moreover, it has been found that when a heterogeneous catalyst is used as a catalyst and ammonia or the like as a base, an ammonium salt of a desired product is obtained only by removing the catalyst from the reaction solution after the reaction through filtration and concentrating the reaction solution thus obtained.

In addition, it has been found that when this process is applied to the production of monatins, high-purity desired products are obtained as crystals by a safe and simple (conventional) procedure.

In the present invention, the novel process for conversion of dihydroisoxazole derivatives to γ-hydroxyamino acid derivatives and the process for producing monatins expected to be useful as sweeteners by this process have been found. These findings have led to the completion of the present invention. According to this process, monatins can easily be obtained without using toxic sodium amalgam as performed by C. W. Holzapfel et al. and without employing the severe deprotection method by heat-refluxing with the strong acid as performed by V. Helaine et al.

That is, the present invention relates to a process for the conversion of dihydroisoxazole derivatives to γ-hydroxyamino acid derivatives through a catalytic hydrogenation reaction, and especially to an efficient process for the production of monatins.

As will be later described in detail, the present invention resides in a process for producing γ-hydroxyamino acid derivatives, especially monatins from dihydroisoxazole derivatives by a catalytic hydrogenation reaction under a specific condition.

### EMBODIMENTS OF THE INVENTION

The embodiments for the present invention are described in detail below.

[1] The present invention resides in a process for producing a γ-hydroxyamino acid derivative represented by the following general formula (2), which comprises subjecting a dihydroisoxazole derivative represented by the following general formula (1) to a catalytic hydrogenation reaction 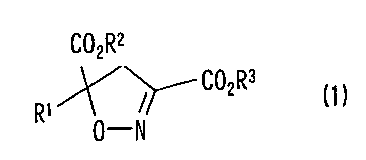 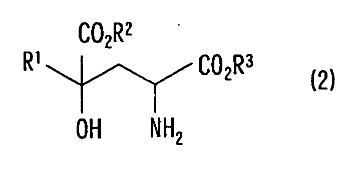 wherein
R¹ represents a 3-indolylmethyl group which may be substituted by at least one of a halogen atom, a hydroxyl group, an alkyl group having up to 3 carbon atoms, an alkoxy group having up to 3 carbon atoms and an amino group,
R² and R³ are independent from each other, and each represents a hydrogen atom, an alkyl group having up to 5 carbon atoms, or an aralkyl group having up to 12 carbon atoms, and
a steric configuration of an asymmetric carbon atom may be in any form of (R), (S) and (RS).

When the form of salt(s) is possible, for example, R² and/or R³ is a hydrogen atom, the dihydroisoxazole derivative and/or the γ-hydroxyamino acid derivative may take the form of salt(s)

In the above described formulas, as R¹, the indole ring in the substituent group may have at least one of a halogen atom (such as an iodine atom, a bromine atom, a chlorine atom, a fluorine atom or the like), a hydroxyl group, an alkyl group having up to 3 carbon atoms, an alkoxy group having up to 3 carbon atoms and an amino group.

R¹ being a 3-indolylmethyl group, it is advantageous for the production of monatins.

In the catalytic hydrogenation reaction, the catalyst can be employed and selected from rhodium catalysts such as a rhodium-active carbon catalyst and a rhodium-alumina catalyst, palladium catalysts such as a palladium-active carbon catalyst and a palladium chloride catalyst, ruthenium catalysts such as a ruthenium-active carbon catalyst, nickel catalysts such as a Raney nickel and platinum catalysts such as a platinum-active carbon catalyst.

It is advisable to use a solvent in the catalytic hydrogenation reaction, and the type of the solvent is not particularly limited so long as it is inactive to the reaction. Water, an alcohol (methanol, ethanol, propanol, etc.), and a mixed solvent of water and alcohol(s) can preferably be used. As the alcohol, a mixture of plural alcohols can naturally be used.

The catalytic hydrogenation reaction can be conducted in the presence or absence of a base. When a base is a used, organic and/or inorganic base(s) such as ammonia, amines, sodium hydroxide, potassium hydroxide, sodium carbonate and sodium bicarbonate can be used.

When water is used as a solvent, a pH value of the solution is preferably in the alkaline condition. The pH value is more preferably on the order of from 8 to 14.

The hydrogenation reaction is preferably conducted in a hydrogen atmosphere. At this time, a hydrogen pressure is not particularly limited. It is preferably from approximately 0.1 to 10 MPa, more preferably from approximately 0.1 to 5 MPa, and further preferably from approximately 0.3 to 5 MPa.

Those skilled in the art can freely determine a stirring efficiency, a reaction temperature, an amount of the catalyst employed and the like in the catalytic hydrogenation reaction. The reaction can be conducted preferably at from approximately - 20 to 100 °C, and more preferably at from approximately 0 to 70 °C.

[2] Monatin(s) [4-hydroxy-4-(3-indolylmethyl)-glutamic acid (s) (including that or those in the salt form(s))] represented by the following structural formula (3) can be produced by utilizing the process described in [1] for the present invention.

Specifically, in the process for producing a γ-hydroxyamino acid derivative in the present invention, a monatin (including that in the salt form) represented by the following structural formula (3) can be produced by selecting a 3-indolylmethyl group as R¹, a hydrogen atom as R² and a hydrogen atom as R³ respectively in the formulas.

The dihydroisoxazole derivative represented by the formula (1) may take the form of salt.

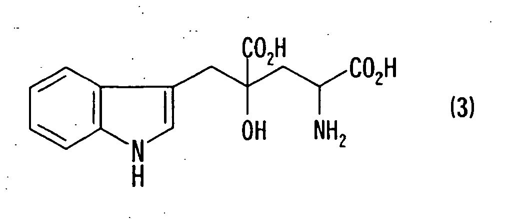

Monatin(s) (including that or those in the salt form (s) ) are obtained by or via the process of the present invention (process for producing a γ-hydroxyamino acid derivative) in the form of a stereoisomer (optical isomer) or in the form of a mixture of plural optical isomers. Even a mixture of plural optical isomers can be used directly as a sweetening ingredient or purified optically by known optical resolution method or the like. Monatin (s) obtained by such purification or analogs thereof (including that or those in the salt form(s)) are also included naturally in those produced by the process of the present invention.

In the process of the present invention, starting materials may be in the form of free compounds or salts, and both of them can be employed in the reaction used in the present invention. Likewise, the desired products produced in the present invention may be in the form of free compounds or salts. When the desired products are produced in the form of salts as a result of the reaction in the present invention, they can be obtained in the form of salts as such or can easily be obtained in the form of free compounds by further conducting an ordinary desalting step (free form-forming step from salt). Meanwhile, when the desired products are produced in the form of free compounds, they can be obtained in the form of free compounds or in the form of salts by further conducting an ordinary salt-forming step. These are all included in the present invention.

The bases used to form salts are not particularly limited. For example, inorganic bases such as sodium hydroxide, potassium hydroxide, calcium hydroxide and sodium carbonate, and organic bases such as ammonia and amines are available.

### EXAMBLES

The present invention is illustrated specifically below by referring to Examples. However, the invention is not limited at all to the Examples.

¹H-NMR spectrum was measured by Bruker AVANCE 400 (400 MHz) and MS spectrum was measured by Thermo Quest TSQ 700 respectively.

### (Example 1)

### Synthesis of diethyl 5-(RS)-(3-indolylmethyl)-4,5 dihydroisoxazole-3,5-dicarboxylate

The above-referenced compound was obtained as a light yellow solid in a total yield of 66 % by using the method described in the foregoing document of C. W. Holzapfel et al. with slight modification.

### (NMR spectrum)

¹H-NMR (CDCl₃, 400 MHz) δppm: 1.24 (3H, t), 1.30 (3H, t) , 3.20 (1H, d), 3.46 (2H, dd), 3.61 (1H, d), 4.14-4.28 (2H, m), 7.11-7.21 (3H, m), 7.38 (1H, d), 7.60 (1H, d), 8.23 (1H, br, s).

### (Example 2)

### Synthesis of 5-(RS)-(3-indolylmethyl)-4,5-dihydroisoxazole-3,5-dicarboxylic acid

1.03 g (3.0 mmols) of the foregoing diethyl ester was dissolved in a mixed solvent of 16 ml of ethanol and 4 ml of water. 290 mg (6.9 mmols) of lithium hydroxide monohydrate was added thereto, and the mixture was stirred at room temperature for 1 hour. The reaction solution was concentrated to a volume of 1/3 or so under reduced pressure, and 15 ml of water and 1N hydrochloric acid were added to adjust the reaction solution to pH of from 1 to 2. The resulting solution was extracted three times with 20 ml of ethyl acetate, and the organic layer was washed with a saturated aqueous solution of sodium chloride, and dried over anhydrous magnesium sulfate. Anhydrous magnesium sulfate was filtered off, and the filtrate was concentrated under reduced pressure to obtain 5-(RS)-(3-indolylmethyl)-4,5-dihydroisoxazole-3,5-dicarboxylic acid as a light yellow powder.

### (NMR spectrum)

¹H-NMR (DMSO-d₆, 400 MHz) ^{δ}ppm: 3.23 (1H, d) , 3.34 (2H, s), 3.43 (1H, d), 6.98 (1H, t), 7.06 (1H, t), 7.19 (1H, d), 7.33 (1H, d), 7.57 (1H, d), 10.98 (1H, s).

### (Example 3)

### Synthesis of monatin

380 mg (1.31 mmols) of the foregoing dicarboxylic acid was dissolved in 8 ml of 28 % aqueous ammonia solution, 200 mg of a 5 % rhodium-active carbon catalyst was added thereto, and a catalytic hydrogenation reaction was conducted under a hydrogen pressure of 1 MPa for 16 hours. The catalyst was filtered off, and the filtrate was freeze-dried to obtain 336 mg of monatin and a small amount of DL-alanine as a byproduct.

### (MS spectrum)

ESI-MS: 291 (M-H).

### (NMR spectrum)

¹H-NMR (400 MHz, D₂O) ^{δ}ppm:
(2S,4S) and (2R,4R) monatin ammonium salts
1.96 (1H, dd, J=11.8 Hz, J=15.2 Hz), 2.57 (1H, dd, J=1.9 Hz, J=15.2 Hz), 3.00 (1H, d, J=14.6 Hz), 3.20 (1H, d, J=14.6 Hz), 3.54 (1H, d, J=10.2 Hz), 7.04 (1H, t, J=7.2 Hz), 7.10 (1H, t, J=7.2 Hz), 7.10 (1H, s), 7.38 (1H, d, J=8.0 Hz), 7.62 (1H, d, J=8.0 Hz).
(2S,4R) and (2R,4S) monatin ammonium salts
2.11 (1H, dd, J=10.4 Hz, J=15.0 Hz), 2.37 (1H, d, J=15.4 Hz), 3.13 (2H, s), 3.88 (1H, d, J=9.8 Hz), 7.05 (1H, d, J=7.6 Hz), 7.14 (2H, s), 7.38 (1H, d, J=7.9 Hz), 7.63 (1H, d, J=7.9 Hz).

### (Example 4)

### Synthesis of monatin

404 mg (1.40 mmols) of the foregoing dicarboxylic acid was dissolved in 8 ml of 14% aqueous ammonia solution, 250 mg of a 5 % rhodium-active carbon catalyst was added thereto, and a catalytic hydrogenation reaction was conducted under a hydrogen pressure of 1 MPa for 7 hours. The catalyst was filtered off, and the filtrate was freeze-dried to obtain 378 mg of monatin and a small amount of DL-alanine as a byproduct.

### EFFECT OF THE TNVENTION

As has been thus far described, the present invention can easily convert dihydroisoxazole derivatives to γ-hydroxyamino acid derivatives in high yield and further can easily produce monatins, one type of γ-hydroxyamino acid derivatives. Accordingly, the present invention can provide γ-hydroxyamino acid derivatives important as various synthetic intermediates, and monatins; materials with sweet taste having excellent properties especially as sweeteners or the ingredients thereof.

Thus, the present invention is quite useful industrially and particularly in the field of foods and pharmaceuticals.

## Claims

1. A process for producing a γ-hydroxyamino acid derivative represented by the following general formula (2), which comprises subjecting a dihydroisoxazole derivative represented by the following general formula (1) to a catalytic hydrogenation reaction 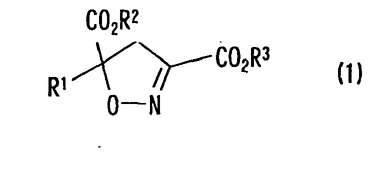 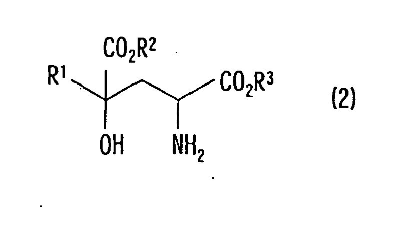 wherein
R¹ represents a 3-indolylmethyl group which may be substituted by at least one of a halogen atom, a hydroxyl group, an alkyl group having up to 3 carbon atoms, an alkoxy group having up to 3 carbon atoms and an amino group,
R² and R³ are independent from each other and each represents a substituent group selected from a hydrogen atom, an alkyl group having up to 5 carbon atoms, and an aralkyl group having up to 12 carbon atoms, and
a steric configuration of an asymmetric carbon atom may be in any form of (R), (S) and (RS),
provided when the form of salt(s) is possible, the dihydroisoxazole derivative and/or the γ-hydroxyamino acid derivative may take the form of salt(s).

2. The process as claimed in claim 1, wherein the catalytic hydrogenation reaction is conducted in the presence of at least one catalyst selected from a rhodium catalyst, a palladium catalyst, a ruthenium catalyst, a nickel catalyst and a platinum catalyst.

3. The process as claimed in claim 1 or 2, wherein the catalytic hydrogenation reaction is conducted in the presence or absence of a base in a solvent selected from a water, an alcohol and a water-alcohol mixed solvent.

4. The process as claimed in any one of claims 1 to 3, wherein the catalytic hydrogenation reaction is conducted at a hydrogen pressure of from 0.1 to 5 MPa.

5. The process as claimed in any one of claims 1 to 4, wherein in the formulas, R¹ is a 3-indolylmethyl group, R² is a hydrogen atom and R³ is a hydrogen atom, and a monatin (which may be in the salt form) represented by the following structural formula (3) is produced, 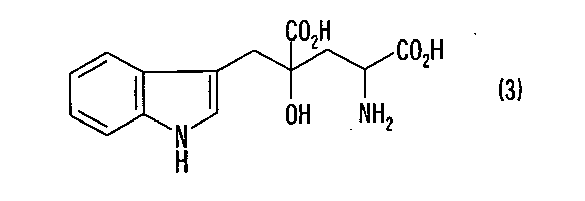 provided the dihydroisoxazole derivative represented by the formula (1) may take the form of salt.

## Patentansprüche

1. Verfahren zur Herstellung eines γ-Hydroxyaminosäurederivats der folgenden allgemeinen Formel (2), welches die katalytische Hydrierungsreaktion eines Dihydroisoxazolderivats der folgenden allgemeinen Formel (1) umfasst, 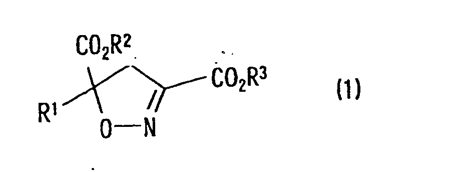 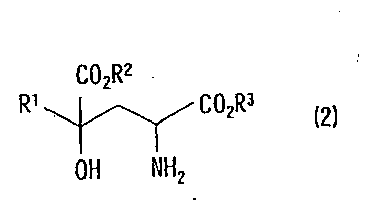 worin
R¹ eine 3-Indolylmethylgruppe darstellt, die mit mindestens einer Gruppe substituiert sein kann, die aus einem Halogenatom, einer Hydroxygruppe, einer Alkylgruppe mit bis zu 3 Kohlenstoffatomen, einer Alkoxygruppe mit bis zu 3 Kohlenstoffatomen und einer Aminogruppe besteht,
R² und R³ unabhängig voneinander jeweils eine Substituentengruppe darstellen, die unter einem Wasserstoffatom, einer Alkylgruppe mit bis zu 5 Kohlenstoffatomen und einer Aralkylgruppe mit bis zu 12 Kohlenstoffatomen ausgewählt ist,
eine sterische Konfiguration eines asymmetrischen Kohlenstoffatoms (R), (S) und (RS) sein kann,
mit der Maßgabe, daß wenn die Form eines oder mehrerer Salze möglich ist, das Dihydroisoxazolderivat und/oder das γ-Hydroxyaminosäurederivat die Form eines oder mehrerer Salze annehmen kann.

2. Verfahren nach Anspruch 1, wobei die katalytische Hydrierungsreaktion in Anwesenheit mindestens eines Katalysators durchgeführt wird, der unter einem Rhodiumkatalysator, einem Palladiumkatalysator, einem Rutheniumkatalysator, einem Nickelkatalysator und einem Platinkatalysator ausgewählt ist.

3. Verfahren nach Anspruch 1 oder 2, wobei die katalytische Hydrierungsreaktion in Anwesenheit oder Abwesenheit einer Base in einem Lösungsmittel durchgeführt wird, das unter Wasser, einem Alkohol und einem Wasser-Alkohol-Mischlösungsmittel ausgewählt ist.

4. Verfahren nach einem der Ansprüche 1 bis 3, wobei die katalytische Hydrierungsreaktion bei einem Wasserstoffdruck von 0,1 bis 5 MPa durchgeführt wird.

5. Verfahren nach einem der Ansprüche 1 bis 4, wobei in den Formeln R¹ eine 3-Indolylmethylgruppe, R² ein Wasserstoffatom und R³ ein Wasserstoffatom darstellen, und wobei ein Monatin (das in der Salzform vorliegen kann) der folgenden Strukturformel (3) hergestellt wird, 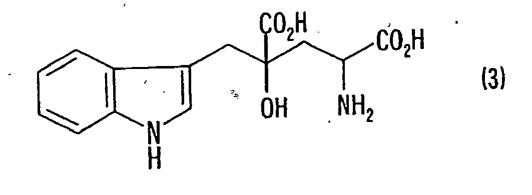 mit der Maßgabe, daß das Dihydroisoxazolderivat der Formel (1) in der Form eines Salzes vorliegen kann.

## Revendications

1. Procédé servant à produire un dérivé de γ-hydroxyaminoacide représenté par la formule générale (2) suivante, lequel comprend de soumettre un dérivé de dihydroisoxazole représenté par la formule générale (1) suivante à une réaction d'hydrogénation catalytique 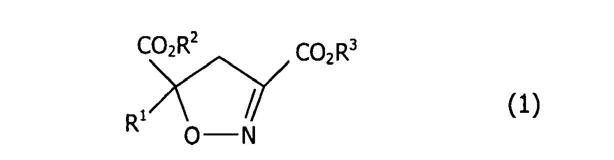 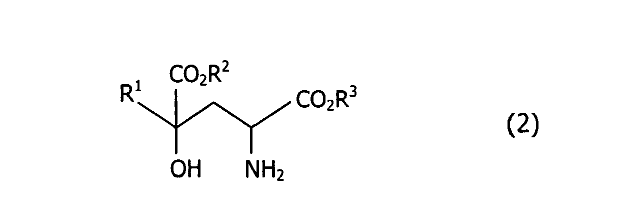 dans lesquelles
R¹ représente un groupe 3-indolylméthyl lequel peut être substitué par au moins l'un d'un atome d'halogène, d'un groupe hydroxyle, d'un groupe alkyle ayant jusqu'à 3 atomes de carbone, d'un groupe alcoxy ayant jusqu'à 3 atomes de carbone et d'un groupe amino,
R² et R³ sont indépendants l'un de l'autre et chacun représente un groupe substituant choisi entre un atome d'hydrogène, un groupe alkyle ayant jusqu'à 5 atomes de carbone et un groupe aralkyle ayant jusqu'à 12 atomes de carbone et
la configuration stérique d'un atome de carbone asymétrique peut être sous l'une quelconque des formes (R), (S) et (RS),
à condition que lorsque la forme de sel(s) est possible, le dérivé de dihydroisoxazole et/ou le dérivé de γ-hydroxyaminoacide puissent prendre la forme de sel(s).

2. Procédé selon la revendication 1, dans lequel la réaction d'hydrogénation catalytique est effectuée en présence d'au moins un catalyseur choisi entre un catalyseur au rhodium, un catalyseur au palladium, un catalyseur au ruthénium, un catalyseur au nickel et un catalyseur au platine.

3. Procédé selon la revendication 1 ou 2, dans lequel la réaction d'hydrogénation catalytique est effectuée en présence ou en l'absence d'une base dans un solvant choisi entre l'eau, un alcool et un solvant mélangé eau-alcool.

4. Procédé selon l'une quelconque des revendications 1 à 3, dans lequel la réaction d'hydrogénation catalytique est effectuée sous une pression d'hydrogène allant de 0,1 à 5 MPa.

5. Procédé selon l'une quelconque des revendications 1 à 4, dans lequel dans les formules, R¹ est un groupe 3-indolylméthyle, R² est un atome d'hydrogène et R³ est un atome d'hydrogène et on produit une monatine (laquelle peut être sous la forme de sel) représentée par la formule de structure (3) suivante, 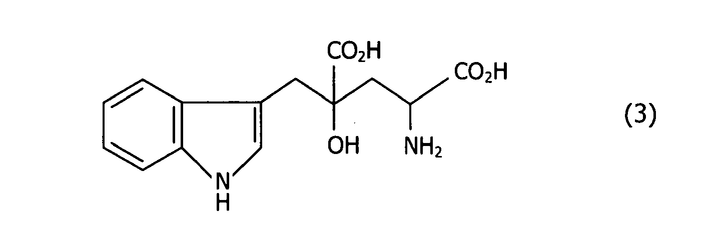 à condition que le dérivé de dihydroisoxazole représenté par la formule (1) puisse prendre la forme de sel.
